# EUROPÄISCHE PATENTSCHRIFT

(11) **EP 2 013 352 B1**
(45) Veröffentlichungstag und Bekanntmachung des Hinweises auf die Patenterteilung: **05.08.2009**
(21) Anmeldenummer: 07728317.4
(22) Anmeldetag: 19.04.2007
(51) Int. Cl.: C12P 7/02

(54) **VERFAHREN ZUR RACEMISIERUNG OPTISCH AKTIVER SEKUNDÄRER ALKOHOLE UNTER VERWENDUNG VON ALKOHOLDEHYDROGENASEN**
PROCESS FOR THE RACEMIZATION OF OPTICALLY ACTIVE SECONDARY ALCOHOLS WITH THE USE OF ALCOHOL DEHYDROGENASE
PROCÉDÉ DE RACÉMISATION D'ALCOOLS SECONDAIRES OPTIQUEMENT ACTIFS EN UTILISANT DES ALCOOL-DÉSHYDROGÉNASES

(30) Priorität: 21.04.2006 EP 06112908
(43) Veröffentlichungstag der Anmeldung: 14.01.2009
(73) Patentinhaber: BASF SE, 67056 Ludwigshafen (DE)
(72) Erfinder: STÜRMER, Rainer, 67127 Rödersheim-Gronau (DE); KROUTIL, Wolfgang, 8042 Graz (AT); FABER, Kurt, 8010 Graz (AT); GRUBER, Christian, 8010 Graz (AT)
(86) Internationale Anmeldenummer: PCT/EP2007/053858
(87) Internationale Veröffentlichungsnummer: WO 2007/122182

(56) Entgegenhaltungen:
- WO-A-20/06021885
- STRAUSS, U.T. ET AL.: "Biocatalytic transformation of racemates into chiral building blocks in 100% chemical yield and 100% enantiomeric excess" TETRAHEDRON: ASYMMETRY, Bd. 10, Nr. 1, 15. Januar 1999 (1999-01-15), Seiten 107-117, XP004156864
- HEIDLAS, J. ET AL.: "Enantioselectivities of enzymes involved in the reduction of methylketones by Baker's yeast" ENZYME AND MICROBIAL TECHNOLOGY, Bd. 13, Nr. 10, 1991, Seiten 817-821, XP002443754

## Beschreibung

Die vorliegende Erfindung betrifft ein Verfahren zur enzymatischen Racemisierung von optisch aktiven sekundären Alkoholen.

### Stand der Technik

Sekundäre Alkohol Dehydrogenasen katalysieren die Oxidation sekundärer Alkohole bzw. die Reduktion des entsprechenden Ketons zum Alkohol.

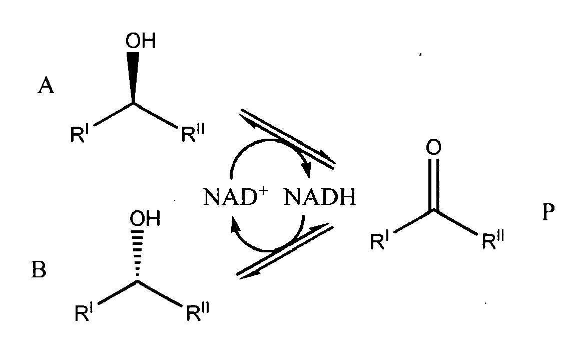

Die vorliegende Erfindung betrifft ein Verfahren zur Racemisierung von optisch aktiven sekundären Alkoholen durch Inkubation dieser Alkohole mit mindestens einer Alkohol-Dehydrogenase der Klasse E.C. 1.1.1.

Als optisch aktive sekundäre Alkohole könne in dem erfindungsgemäßen Verfahren eine Vielzahl von strukturell verschiedenen Alkoholen bzw. sekundäre Alkoholgruppen enthaltende Verbindungen eingesetzt werden.

Geeignet sind aliphatische Alkohole mit einer Kettenlänge von 4 bis ca. 20 C-Atomen, wobei der aliphatische Rest verzweigt oder unverzweigt, ein- oder mehrfach ungesättigt oder auch zyklisch sein kann oder Teile eines zyklischen Systems bilden kann, beispielsweise eines heterozyklischen Systeme wie Morpholin, Pyrrol, Thiophen, Pyrazol, Imidazol, Oxazol, Thiazol, Pyridin, Pyran, Pyrimidin, Pyridazin, Pyrazin, Cumaron, Indol , Chinolin.

Bevorzugte aliphatische Alkohole sind 2- Butanol, 2- Pentanol, 2- Hexanol, 3- Hexanol, 2-Heptanol, 3-Heptanol, 2-Oktanol, 3-Oktanol, 2-Nonanol, 2-Dekanol.

Weitere geeignete sekundäre Alkohole sind solche die eine Aryl-Alkyl-Struktur besitzen, wobei der aromatische Teil homoaromatisch oder heteroaromatisch sein kann.

Bevorzugte Alkohole sind solche die als aromatischen Teil eine ggf. substituierte Phenyl, Naphtyl, Pyridyl Gruppe tragen.

Besonders bevorzugte sekundäre Alkohole sind ggf. substituiertes Phenyl-Ethanol-2, Phenyl-propanol-2, Phenyl-butanol-2, Phenyl-pentanol-2, Phenyl-hexanol-2.

Die Alkohole können auch ein oder mehrfach substituiert sein, d.h. ein oder mehrere H-Atome sind durch Gruppen wie F, Cl, Br, J, NH₂, NHR, NR₂, SH, CN, COOH, COOR, CO, CS, CNH, NO₂, worin R für Alkyl oder Alkylarylreste stehen kann.

Als Alkohol-Dehydrogenase geeignet sind NAD-oder NADP-abhängige Oxidoreduktasen der Enzymklassifikation E.C. 1.1.1, insbesondere Alkoholdehydrogenasen, bevorzugt solche, die aus Mikroorganismen isoliert wurden oder aus solchen mittels gentechnischer Verfahren isoliert und/oder verändert wurden. Die Veränderung kann entweder durch sog. Random-mutagenesis Verfahren oder durch sog. site-specific mutagenesis eingeführt werden

Als bevorzugte Alkohol-Dehydrogenasen werden solche aus Mikroorganismen der Gattung Lactobacillus, insbesondere der Art Lactobacillus kefir und solche der Gattung Rhodococcus, insbesondere der Art Rhodococcus erythropolis verwendet.

Diese Alkohol-Dehydrogenasen sind kommerziell erhältlich (z.B. von Fluka) oder aus öffentlich zugänglichen Stammsammlungen zu beschaffen und mit bekannten Methoden zu isolieren. Beispielsweise Lactobacillus kefiri DSM 20587, ATCC 35411; Rhodococcus erythropolis DSM 43066, ATCC 25544.

Als Alkohol-Dehydrogenasen werden in einer bevorzugten Ausführungsform mehrere verschiedene Alkohol-Dehydrogenasen verwendet, bevorzugt solche, die eine unterschiedliche Prelog Spezifität besitzen, besonders bevorzugt eine Alkohol-Dehydrogenase mit Prelog-Spezifität und eine zweite Alkohol-Dehydrogenase mit anti-Prelog-Spezifität. Hinsichtlich der Definition von Prelog-Spezifität wird auf das Dokument Kurt Faber, Pure Appl. Chem. 69, 1613-1632, 1997 , insbesondere auf das Kapitel Redoxreaktionen, verwiesen, das hiermit ausdrücklich in Bezug genommen wird.

Aus thermodynamischen Überlegungen kann jede Alkohol-Dehydrogenase racemisieren. Die zur Racemisierung eingesetzte Alkohol-Dehydrogenase sollte sowohl für die Oxidation als auch für die Reduktion eine möglichst geringe Enantio/Stereo-Selektivität aufweisen um eine möglichst hohe Racemisierungs-Geschwindigkeit zu erreichen. Da die Selektivität u.A. eine Funktion der Temperatur ist, kann bei höherer Temperatur mit einem unter normal' Bedingungen selektivem Enzym (z.B. *L. kefir* Alkohol-Dehydrogenase) ebenfalls racemisiert werden, wenn die Aktivität unter diesen,Bedingungen nicht verloren geht. Alkohol-Dehydrogenasen mit einer hohen Enantio- und Stereoselektivität (E>200) zeigen eine langsame Racemisierungs-Aktivität.

Bei Zwei- oder Mehr-Enzym-Systemen setzt sich die Selektivität des Alkohol-Dehydrogenase Systems aus den Selektivitäten der eingesetzten Enzyme zusammen. Durch gleichzeitigen Einsatz eines 'Prelog'-Enzyms (*R*. *erythropolis* Alkohol-Dehydrogenase) und eines anti-,Prelog'-Enzyms (*L. kefir* Alkohol-Dehydrogenase), die jeweils eine hohe Substrat-bezogene Enantio- bzw. Stereoselektivität aufweisen, jedoch gegenläufige Stereo-Präferenz haben, liegt die Gesamt-Selektivität des Systems bei 1 und die Racemisierung wird beschleunigt.

Die erfindungsgemäß verwendeten Enzyme mit Dehydrogenase-Aktivität können im erfindungsgemäßen Verfahren als freies oder immobilisiertes Enzym verwendet werden.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Das erfindungsgemäße Verfahren kann je nach Substrat in einem zusätzlichen Lösungsmittel oder im Substrat selbst als Lösungsmittel durchgeführt werden. Als Lösungsmittel kommen alle gängigen organischen Lösungsmittel in Betracht, die eine enzymatische Redoxreaktion gestatten, insbesondere Alkohole, Ketone, Ether, Kohlenwasserstoffe oder Gemische dieser Stoffe. Vorteilhafterweise werden solche Lösungsmittel gewählt, die eine leichte Abtrennung des racemischen sekundären Alkohls erlauben.

Unter optisch aktiven Alkoholen sind im erfindungsgemäßen Verfahren Enantiomere zu verstehen, die eine Enantiomerenanreicherung zeigen. Bevorzugt werden im Verfahren Enantiomerenreinheiten von mindestens 70 %ee, bevorzugt von min. 80 %ee, besonders bevorzugt von min. 90 %ee, ganz besonders bevorzugt min. 98 %ee verwendet.

Unter Racemisierung von optisch aktiven Alkoholen wird hier eine Verringerung der Enantiomerenreinheit gegenüber dem Ausgangsalkohol (Substrat) verstanden, insbesondere eine Verringerung der Enantiomerenreinheit um 10, 20, 25, 30 Prozentpunkte. Racemisierung ist nicht in der Weise zu verstehen, dass zwangsläufig ein Enantiomerenverhältnis von 50:50 erreicht werden muss, obwohl diese vollständige Racemisierung eine bevorzugte Ausführungsform der Erfindung darstellt.

Die eingesetzte Menge an Cofaktor (NAD/NADH bzw. NADP/NADPH) ist für die Racemisierung unerheblich,solange sichergestellt ist, dass pro Mol Enzym mind. ein Mol NAD⁺ bzw. NADP vorhanden ist, um die vollständige Bildung des Enzym-Cofaktor Komplexes sicherzustellen. Die Konzentration an intermediär gebildetem Keton wird durch das eingesetzte NAD⁺/NADH Verhältnis sowie durch das Redox-potential Alkohol/Keton reguliert.

Das erfindungsgemäße Verfahren kann auch vorteilhaft mit Systemen zur Cofaktor-Regeneration gekoppelt werden.

Das erfindungsgemäße Verfahren kann sowohl kontinuierlich als auch diskontinuierlich betrieben werden.

### Experimenteller Teil

### Verwendete Alkohole (Substrat)

| **Nummer** | **Bezeichnung** | **Struktur** |
|---|---|---|
| **1** | 2-Octanol | 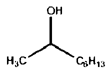 |
| **2** | 2-Heptanol | 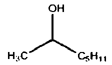 |
| **3** | 2-Nonanol | 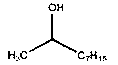 |
| **4** | 1-Phenyl-2-propanol | 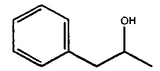 |
| **5** | 6-Methyl-5-hepten-2-ol | 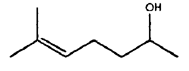 |

### Verwendete Enzyme

| **Abk.** | **Bezeichnung** | **Quelle** |
|---|---|---|
| **LK-Alkohol-** | Alkohol-Dehydrogenase *Lactobacillus* | Kommerziell |
| **Dehydrogenase** | *kefir* | Fluka 05643 |
| **RE-Alkohol-** | Alkohol-Dehydrogenase *Rhodococcus* | Kommerziell |
| **Dehydrogenase** | *erythropolis* | Jülich Fine Chemicals 04.11 |

### Verwendete Enzyme

| **Enzym Stammlösungen** | **Konzentration** | **U/mL** |
|---|---|---|
| **LK-Alkohol-Dehydrogenase** | 8mg/mL | 3 |
| **RE-Alkohol-Dehydrogenase** | 80mg/mL | 3 |
| **RE-Alkohol-Dehydrogenase & LK-Alkohol-Dehydrogenase** | 4mg/mL | 1.5 |
| | 40mg/mL | 1.5 |

50µL Enzym-Stammlösung wurden in einem 2mL Eppendorf®-Gefäß in 500µL Phosphat Buffer (50mM, pH 7.5) gelöst und 10µL NAD⁺/NADH Stammlösung (50mg NADH/mL, 30mg NAD⁺/mL) zugegeben. Danach wurde die Reaktion durch Zugabe von 2µL Substrat gestartet (48h, 30°C/65°C, 130rpm). Die Reaktion wurde durch Extraktion mit 500µl EtOAc gestoppt.

### Ein-Enzym System

| **Substrat** | **e.e. Substrat^{a} [%]** | **Enzym** | **Temperatur [°C]** | **Keton [%]** | **e.e.**^{a,b} **[%]** |
|---|---|---|---|---|---|
| | | RE-Alkohol-Dehydrogenase | 30 | <0.1 | -98.35 |
| **(*R*)-1** | >-99.9 | LK-Alkohol-Dehydrogenase | 30 | 22.61 | -98.04 |
| | | LK-Alkohol-Dehydrogenase | 65 | <0.1 | -4.53 |
| **(*S*)-1** | >99.9 | RE-Alkohol-Dehydrogenase | 30 | 0.25 | 99.53 |
| | | LK-Alkohol-Dehydrogenase | 30 | 0.00 | >99.9 |
| **(*R*)-2** | >-99.9 | R-Alkohol-Dehydrogenase | 30 | n.d. | <-99.9 |
| | | LK-Alkohol- Dehydrogenase | 30 | n.d. | <-99.9 |
| **(*R*)-3** | >-99.9 | RE-Alkohol-Dehydrogenase | 30 | 1.67 | -97.54 |
| | | LK-Alkohol-Dehydrogenase | 30 | 27.30 | -93.04 |
| **(*S*)-4** | >99.9 | RE-Alkohol-Dehydrogenase | 30 | 3.12 | >99.9 |
| | | LK-Alkohol-Dehydrogenase | 30 | 3.63 | >99.9 |
| **(*R*)-5** | >-99.9 | E-Alkohol-Dehydrogenase | 30 | 0.56 | -98.12 |
| | | LK-Alkohol-Dehydrogenase | 30 | 24.38 | -73.94 |

| | | | | | |
|---|---|---|---|---|---|
| ^{a} Positive bzw. negative e.e. Werte beziehen sich auf den Überschuß des jeweiligen Enantiomers (*S*) bzw. (*R*). ^{b} e.e. nach einer Reaktionszeit von 48h. | | | | | |

### Zwei-Enzym System

| **Substrat** | **e.e. Substrat^{a} [%]** | **Enzym** | **Temperatur [°C]** | **Keton [%]** | **e.e.**^{a,b} **[%]** |
|---|---|---|---|---|---|
| **(*R*)-1** | >-99.9 | RE-Alkohol-Dehydrogenase & LK-Alkohol-Dehydrogenase | 30 | 2.23 | -9.49 |
| **(*S*)-1** | >99.9 | RE-Alkohol-Dehydrogenase & LK-Alkohol-Dehydrogenase | 30 | 3.35 | 36.95 |
| **(*R*)-2** | >-99.9 | RE-Alkohol-Dehydrogenase & LK-Alkohol-Dehydrogenase | 30 | n.d. | -40.21 |
| **(*R*)-3** | >-99.9 | RE-Alkohol-Dehydrogenase & LK-Alkohol-Dehydrogenase | 30 | 2.57 | -32.19 |
| **(*S*)-4** | >99.9 | RE-Alkohol-Dehydrogenase & LK-Alkohol-Dehydrogenase | 30 | 23.12 | >99.9 |
| **(*R*)-5** | >-99.9 | RE-Alkohol-Dehydrogenase. & LK-Alkohol-Dehydrogenase | 30 | 5.74 | -23.92 |

| | | | | | |
|---|---|---|---|---|---|
| ^{a} Positive bzw. negative e.e. Werte beziehen sich auf den Überschuß des jeweiligen Enantiomers (*S*) bzw. (*R*). ^{b} e.e nach einer Reaktionszeit von 48h. | | | | | |

### Analytik

### GC-Analysen

Gas Chromatograph: Varian 3900 Gas Chromatographen (FID)
Säule: Chrompack Chirasil-DEX CB (25m x 0.32mm x 0.25µm, 1.Obar H₂)

| **Substr.** | **GC-Säule** | **Tempereatur-programm^{a}** | **Retentionszeit [min]** | **ee.**^{b} **[%]** |
|---|---|---|---|---|
| | | | (*S*) 1.369 | >99.9 |
| **2** | Chirasil-DEX CB | 110/0/2.5/120/0/10/200/0 | (*R*) 1.434 | <-99.9 |
| | | | Keton n:d. | - |
| | | | (*S*) 2.741 | >99.9 |
| **3** | Chirasil-DEX CB | 110/0/2.5/120/0/10/200/0 | (*R*) 2.988 | <-99.9 |
| | | | Keton 1.795 | - |
| | | | (*S*) 3.683 | >99.9 |
| **4** | Chirasil-DEX CB | 110/0/2.5/120/0/10/200/0 | (*R*) n.d. | <-99.9 |
| | | | Keton 2.65 | - |
| | | | (*S*) 1.860 | >99.9 |
| **5** | Chirasil-DEX CB | 110/0/2.5/120/0/10/200/0 | (*R*) 1.981 | <-99.9 |
| | | | Keton 1.303 | - |

| | | | | |
|---|---|---|---|---|
| ^{a} °C/holding time [min]/heating rate [°C/min]/°C/holding time/heating rate/°C/holding time [min], ^{b} Positive bzw. negative e.e. Werte beziehen sich auf den Überschuß des jeweiligen Enantiomers (S) bzw. (R). n.d.: nicht detektierbar Acetylierung von Alkoholen (Generelle Methode) | | | | |

Die Derivatisierung wurde durch Zugabe von 100µl Acetanhydrid/DMAP Lösung und Inkubation bei 30°C/130rpm für 60min durchgeführt. 0.5mL H20 wurden hinzugefügt. Die organische Phase wurde abgehoben und über Na2SO4 getrocknet.

## Patentansprüche

1. Verfahren zur Racemisierung von optisch aktiven sekundären Alkoholen durch Inkubation dieser Alkohole mit mindestens einer Alkohol-Dehydrogenase der Klasse E.C. 1.1.1.

2. Verfahren nach Anspruch 1, **dadurch gekennzeichnet, dass** als Alkohol-Dehydrogenase eine mikrobielle Alkohol-Dehydrogenase verwendet wird.

3. Verfahren nach Anspruch 1, **dadurch gekennzeichnet, dass** als Alkohol-Dehydrogenase eine Alkohol-Dehydrogenase aus einem Mikroorganismus der Gattung Lactobacillus oder Rhodococcus verwendet wird.

4. Verfahren nach Anspruch 1, **dadurch gekennzeichnet, dass** als Alkohol-Dehydrogenase eine Mischung von zwei Alkohol-Dehydrogenasen verwendet wird.

5. Verfahren nach Anspruch 4, **dadurch gekennzeichnet, dass** die eine Alkohol-Dehydrogenase eine Prelog-Spezifität aufweist und die andere Alkohol-Dehydrogenase eine anti-Prelog Spezifität aufweist.

6. Verfahren nach Anspruch 1, **dadurch gekennzeichnet dass** die Inkubation in Abwesenheit von organischen Lösungsmitteln stattfinden kann.

## Claims

1. A method for racemizing optically active secondary alcohols by incubating these alcohols with at least one alcohol dehydrogenase of the E.C. 1.1.1. class.

2. The method according to claim 1, wherein a microbial alcohol dehydrogenase is used as alcohol dehydrogenase.

3. The method according to claim 1, wherein an alcohol dehydrogenase from a microorganism of the genus Lactobacillus or Rhodococcus is used as alcohol dehydrogenase.

4. The method according to claim 1, wherein a mixture of two alcohol dehydrogenases is used as alcohol dehydrogenase.

5. The method according to claim 4, wherein one alcohol dehydrogenase has a Prelog specificity and the other alcohol dehydrogenase has an anti-Prelog specificity.

6. The method according to claim 1, wherein the incubation can take place in the absence of organic solvents.

## Revendications

1. Procédé pour la racémisation d'alcools secondaires optiquement actifs par incubation de ces alcools avec au moins une alcool-déshydrogénase de la classe E.C. 1.1.1.

2. Procédé selon la revendication 1, **caractérisé en ce qu'**on utilise comme alcool-déshydrogénase une alcool-déshydrogénase microbienne.

3. Procédé selon la revendication 1, **caractérisé en ce qu'**on utilise comme alcool-déshydrogénase une alcool-déshydrogénase provenant d'un microorganisme du genre Lactobacillus ou Rhodococcus.

4. Procédé selon la revendication 1, **caractérisé en ce qu'**on utilise comme alcool-déshydrogénase un mélange de deux alcool-déshydrogénases.

5. Procédé selon la revendication 4, **caractérisé en ce que** l'une alcool-déshydrogénase présente une spécificité Prelog et l'autre alcool-déshydrogénase présente une spécificité anti-Prelog.

6. Procédé selon la revendication 1, **caractérisé en ce que** l'incubation peut avoir lieu en l'absence de solvants organiques.
